# EUROPÄISCHE PATENTSCHRIFT

(11) **EP 1 038 484 B1**
(45) Veröffentlichungstag und Bekanntmachung des Hinweises auf die Patenterteilung: **05.10.2005**
(21) Anmeldenummer: 00100902.6
(22) Anmeldetag: 18.01.2000
(51) Int. Cl.: A47K 3/02, A47K 3/00

(54) **Badewanne mit einem beleuchteten Innenraum**
Bathtub with illuminated interior
Baignoire avec intérieur éclairé

(30) Priorität: 20.03.1999 DE 29905156 U
(43) Veröffentlichungstag der Anmeldung: 27.09.2000
(73) Patentinhaber: Wuschik, Günter, Dipl.-Ing., 63849 Leidersbach (DE)
(72) Erfinder: Wuschik, Günter, Dipl.-Ing., 63849 Leidersbach (DE)
(74) Vertreter: Pöhner, Wilfried Anton, Dr.

(56) Entgegenhaltungen:
- WO-A-99/23413
- DE-A- 4 309 108
- DE-A- 19 747 980
- DE-C- 19 937 874
- US-A- 5 680 730
- PATENT ABSTRACTS OF JAPAN vol. 017, no. 142 (C-1038), 23. März 1993 (1993-03-23) & JP 04 314409 A (MATSUSHITA ELECTRIC WORKS LTD), 5. November 1992 (1992-11-05)

## Beschreibung

Die Erfindung bezieht sich auf eine Badewanne mit einem beleuchteten Innenraum, mit einer in der Wandung der Badewanne montierten Lichtquelle.

Die Beleuchtung des Innenraums einer Badewanne ist bekannt und gebräuchlich. Hierbei kommen unter anderem Glühlampen zur Anwendung, die mit einem geeigneten Gehäuse versehen, direkt in die Wandung der Badewanne eingebaut werden, und das Licht durch eine Scheibe in den Innenraum der Badewanne abstrahlen. Zur Erzeugung des Lichts werden konventionelle Glühlampen oder Halogenlampen verwendet. Eine weitere Möglichkeit der Beleuchtung des Innenraums einer Badewanne besteht darin, daß das von einer räumlich von der Badewanne getrennten Glühlampe erzeugte Licht in eine oder mehrere Lichtleitfasern eingekoppelt wird, und diese stirnseitig in die Wandung der Badewanne als Lichtquellen eingebaut sind. Solche Badewannen mit einem beleuchteten Innenraum werden unter anderem zur therapeutischen Behandlung mit Licht eingesetzt, zum anderen werden sie für den Privatgebrauch verwendet, um dem Nutzer ein subjektives Wohlgefühl und eine neue Art der Entspannung zu bieten.

Der Nachteil bisher bekannter Badewannen mit einem beleuchteten Innenraum besteht darin, daß die Halogenlampen teuer in der Anschaffung und aufgrund ihrer Größe aufwendig in der Montage sind, und wegen der Versorgungsspannung von 230 V trotz umfangreicher Sicherheitsmaßnahmen immer ein Gesundheitsrisiko von ihnen ausgeht. Desweiteren geben Glüh- oder Halogenlampen einen großen Teil der aufgenommenen Energie als Infrarotstrahlung wieder ab, so daß an den Scheinwerferscheiben im Wasser Temperaturen bis zu 80° Celsius auftreten. Der Nachteil einer Glühlampe, die mit Lichtleitfasern gekoppelt, den Innenraum der Badewanne ausleuchtet, besteht darin, daß dieses Prinzip einer aufwendigen und teuren Konstruktion bedarf, und es hierbei praktisch unmöglich ist, Licht beliebiger Farbe in verschiedenen Lichtquellen zu erzeugen. Es ist zwar bekannt, zwischen Lichtleitfaser und Glühlampe einen Farbwechselfilter einzubauen, doch wird hierbei im allgemeinen die Lichtfarbe in allen Lichtleitfasern gleichzeitig verändert.

WO 99 23413 und DE 197 47 980 A1 offenbaren Beleuchtungskörper für Schwimmbäder und ähnliche Feuchträume, die aus einem Einbaugehäuse für Leuchtdioden, einer Stromzufuhr mit transparenten Abdeckdeckeln besteht, wobei vorgesehen ist, dass die Leuchtdioden einzeln oder gruppenweise durch eine Steuerschaltung ansteuerbar und/oder in der Versorgungsspannung getrennt regelbar sind. In Bezug auf die erfindungsgemäße Anmeldung handelt es sich bei beiden Dokumenten um den sogenannten nachveröffentlichten Stand der Technik, der für die Beurteilung der erfinderischen Tätigkeit im Zusammenhang mit der erfindungsgemäßen Anmeldung nicht von Bedeutung ist.

DE 43 091 08 A1 offenbart eine Badewanne deren Innenraum beleuchtet wird, indem Lichtleitfasern, die jeweils am anderen Enden mit einer Lichtquelle, beispielsweise einer Glühlampe verbunden sind, in der Wandung der Badewanne enden.

US-A-5 680 730 beschreibt eine metallische Reling, deren Querschnitt einen zylindrischen Kanal aufweist, der auf der dem Wasser zugewandten Längsseite offen ist und zur Aufnahme eines Lichtleiters (z. B. eines Glasfaserkabels) oder einer Lichtquelle (z. B. einer Licht-Emittierenden-Diode, LED) dient. Die Innenseite des Kanals weist zur Verbesserung der Lichtausbeute im Innenraum des Schwimmbeckens eine Oberfläche mit reflektierenden Eigenschaften auf.

Ausgehend vom Stand der Technik liegt der Erfindung das Problem zugrunde, eine Badewanne mit einem beleuchteten Innenraum so zu gestalten, dass die Erzeugung verschiedenster Beleuchtungseffekte möglich ist, dass die Realisierung eines solchen beleuchteten Badewanneninnenraums in einfacher und kostengünstiger Weise erfolgen kann, und dass mit der Beleuchtung keine Risiken für den Nutzer durch elektrischen Strom verbunden sind.

Dieses Problem wird erfindungsgemäß dadurch gelöst, dass die Lichtquelle eine licht-emittierende-Diode, auch als LED bekannt, ist, und die LED mehrere energetisch verschiedene lichtemittierende Halbleiterübergänge aufweist.

Der Kemgedanke der Erfindung besteht darin, dass der Innenraum der Badewanne mit einer LED beleuchtet wird. In einer licht-emittierenden Diode wird Licht aufgrund der Rekombination von Elektron-Loch-Paaren im Übergangsgebiet eines in Durchlaßrichtung gepolten P-N-Übergangs eines dotierten Halbleiters erzeugt und emittiert. Die Größe der Bandlücke dieses Halbleiters bestimmt im wesentlichen die Wellenlänge und damit die Farbe des Lichts. Es sind heute LEDs zur Erzeugung praktisch jeder Farbe des sichtbaren Lichts in verschiedenster Ausführung erhältlich, mit Lichtstärken bis zu ein Candela. Aufgrund ihrer äußeren Abmessungen, sie haben üblicherweise einen Durchmesser von maximal 10 mm und eine Höhe des Leuchtkörpers, d.h. der den Halbleiter umgebenden transparenten Haube, von ebenfalls 10 mm, sind sie auch in gekrümmten Wandbereichen der Badewanne installierbar. Die genaue Art und Weise wie die LED in die Wandung der Badewanne eingebaut ist, ist erfindungsgemäß unerheblich, kann aber von fachlich geschulten Personen in vielfältiger Weise realisiert werden. Üblicherweise werden diese LEDs mit Spannungen von 2 bis maximal 5 V und Strömen zwischen 10 und 50 mA betrieben, so daß eine Gefährdung einer Person in einer mit Wasser gefüllten Badewanne aufgrund eines Kurzschlusses praktisch ausgeschlossen ist.

Der Vorteil der Erfindung besteht darin, daß die Beleuchtung des Innenraums einer Badewanne mit Hilfe der LEDs in praktisch beliebiger Art realisiert werden kann. Darüberhinaus sind sie günstig in der Anschaffung und es geht kein Gefährdungspotential durch elektrischen Strom von ihnen aus.

Vorteilhafte Ausgestaltungen der Erfindung sind Gegenstand von Unteransprüchen.

Eine sinnvolle Ausgestaltung der Erfindung besteht darin, daß die Badewanne mit mehreren Lichtquellen in der Wandung versehen ist. Die an beliebigen Stellen der Wandung angebrachten Lichtquellen ermöglichen beispielsweise eine gleichmäßige Ausleuchtung des Innenraums der Badewanne und eine Steigerung der Lichtstärke. Desweiteren ist, durch die Ausstattung verschiedener Lichtquellen mit verschiedenen LEDs, die Erzielung beliebiger Lichteffekte möglich. Aufgrund des geringen Gewichts und der Abmessungen einer LED ist es möglich, eine große Anzahl von LEDs in die Wandung einer Badewanne einzubauen, ohne deren Stabilität zu beeinträchtigen.

Die Austattung einer Lichtquelle mit mehreren und/oder verschiedenen LEDs, bietet den Vorteil, daß die Intensität des ausgestrahlten Lichts erhöht wird, oder aber daß von einer Lichtquelle Licht verschiedener Farbe in den Innenraum abgestrahlt wird, so daß verschiedenartige Ausleuchtungen der Badewanne erzielt werden.

Die LED wird so ausgeführt, daß sie mehrere lichtemittierende Halbleiterübergänge mit verschieden großen Bandlücken aufweist. Da die Wellenlänge, wie oben gezeigt, von der Größe der Bandlücke abhängt, kann die LED Licht der jeweiligen Farbe in den Innenraum der Badewanne ausstrahlen. Es ist dem Fachmann möglich, die LED, z. B. mit Hilfe einer geeigneten Steuerungselektronik, so zu beeinflussen, daß nur ein bestimmter oder mehrere Halbleiterübergänge Licht mit einer gewünschten Intensität aussenden. Da Lichtwellen sich ungestört überlagern, ist mit einer solchen LED die Erzeugung von Licht mit verschiedenen Farben, die sich als Kombination der einzelnen Wellenlängen ergeben, möglich.

Das für das menschliche Auge sichtbare Licht kann aus den drei Grundfarben rot, grün und blau kombiniert werden. Eine LED die, wie oben angeführt, über Halbleiterübergänge zur Erzeugung dieser drei Farbkomponenten verfügt, kann Licht jeder Farbe aus dem sichtbaren Spektrum in die Badewanne einstrahlen und ist dem Fachmann bekannt. Dabei wird die Intensität der einzelnen Komponenten durch den an den jeweiligen Halbleiterübergang angelegten Strom beeinflußt, und beliebige Beleuchtungseffekte sind möglich.

Um eine wunschgemäße Ausleuchtung des Badewanneninnenraums zu erreichen, werden die LEDs einzeln und/oder gruppenweise angesteuert. Eine Realisierung einer solchen Steuerung, beispielsweise mit Hilfe einer Steuerelektronik, kann von fachlich geschultem Personal mit Hilfe gebräuchlicher Techniken ausgeführt werden. Damit ist es beispielsweise auch möglich, eine oben angeführte LED so zu steuern, daß sie nacheinander verschiedenfarbiges Licht emittiert. Die Steuerung der LEDs kann auch derart erfolgen, daß in der Badewanne umlaufende Lichtimpulse, farbige Ausleuchtungen verschiedener Bereiche oder eine zufallsgesteuerte Ausleuchtung des Innenraums erfolgt. Insbesondere wird hierbei die Beeinflussung der Beleuchtung durch die sich in der Badewanne befindende Person mit einer beliebig ausgeführten Steuerung vorgeschlagen.

Die Anordnung der Lichtquellen in der Wandung der Badewanne kann erfindungsgemäß in beliebiger Art und Weise erfolgen, doch können beispielsweise mit einer symmetrischen Anordnung der Lichtquellen eindrucksvolle Lichteffekte erzielt werden. Besonders wird hier vorgeschlagen, daß die Lichtquellen äquidistant auf dem Umfang der Badewannenwandung verteilt sind, so daß eine Art Lichtkranz erzeugt wird. Desweiteren können die Lichtquellen auf einer Geraden, z. B. am Boden der Wanne, oder in geometrische Mustern wie Vierecken, Dreiecken oder Kreisen angeordnet sein.

Die Ausführung der Lichtquelle ist erfindungsgemäß unerheblich. Eine Art der Ausgestaltung besteht darin, daß die LED in einer Hülse befestigt, beispielsweise eingeklebt ist, und die Hülse mit einer Schraubverbindung auf ein Gewinde, das an der Wandung der Badewanne befestigt ist, aufgeschraubt wird. Desweiteren besteht eine sinnvolle Ausführung darin, daß eine durchsichtige Scheibe, beispielsweise aus Glas, annähernd bündig in die Badewannenwandung eingepaßt ist und die LED vom Innenraum der mit Wasser gefüllten Badewanne abtrennt. Die notwendige Abdichtung einer solchen Scheibe mit Hilfe einer Gummidichtung ist bekannter Stand der Technik.

Die Lichtquellen können auch so angeordnet werden, daß sie nahe beieinander liegen, d. h. daß ihr gegenseitiger Abstand in der Größenordnung des Durchmessers der Lichtquellen liegt. Mit einem solchen Bündel von Lichtquellen kann die besonders intensive Ausleuchtung eines Bereichs der Badewanne, beispielsweise des Kopfbereichs, erreicht werden.

Eine Änderung der Farbe des von der Lichtquelle ausgehenden Lichts ist auch mit dem Austausch einer LED gegen eine andere erreichbar. Ein solcher Wechsel kann von einer mechanischen Vorrichtung, insbesondere einem Revolverkopf, an dem mehrere LEDs befestigt sind, vorgenommen werden. Die hierzu notwendigen Maßnahmen sind dem Fachmann bekannt. Damit ist auch ein einfacher Zugriff auf eine LED, beispielsweise wenn sie beschädigt ist, möglich.

Eine Ausgestaltung der Erfindung besteht darin, daß die Lichtquelle großflächig ausgeführt wird, was bedeutet, daß das Licht nicht mehr von einer punktförmigen Lichtquelle ausgehend in den Innenraum der Badewanne abgestrahlt, sondern daß das von der LED erzeugte Licht, z. B. mit Hilfe eines Diffusors zwischen LED und Lichtquelle, gestreut und aufgeweitet wird. Besonders wird vorgeschlagen, die großflächige Lichtquelle so auszuführen, daß sie als Streifen bzw. Band in der Badewannenwandung angebracht ist und mehrere LEDs in das Leuchtband eingebaut sind.

Weitere Einzelheiten, Merkmale und Vorteile der Erfindung lassen sich dem nachfolgenden Beschreibungsteil entnehmen, in dem anhand einer Zeichnung ein Ausführungsbeispiel der Erfindung näher erläutert ist. Es zeigen:
- **Figur 1**: den schematischen Querschnitt einer Badewanne mit Lichtquellen in der Wandung.
- **Figur 2**: den Querschnitt einer Lichtquelle.

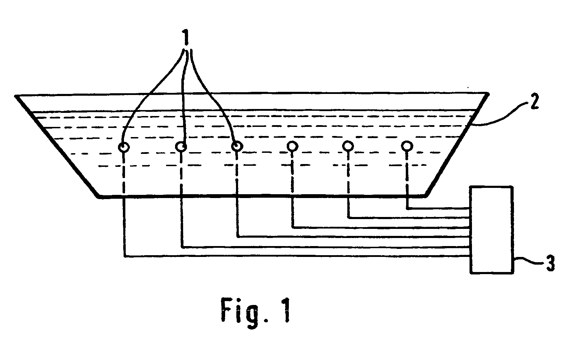

Bei dem in Figur 1 dargestellten Ausführungsbeispiel sind mehrere Lichtquellen (1) äquidistant auf einer Geraden in der Wandung (7) einer Badewanne (2) eingebaut. Die licht-emittierenden Dioden (4) sind mit einer Steuerelektronik (3) verbunden, die auch die Energieversorgung der LEDs (4) übernimmt. Sie ist räumlich von der Badewanne (2) getrennt, so daß durch das Wasser in der Badewanne (2) keine Kurzschlußgefahr entsteht.

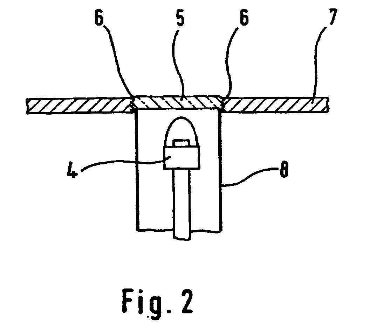

Bei der beispielhaft in Figur 2 dargestellten Lichtquelle (1) ist die LED (4) mit einer durchsichtigen Scheibe (5) vom Innenraum der Badewanne (2) abgetrennt. Die Lichtquelle (1) verfügt hier über eine Verschraubung (6) mit der sie an der Wandung (7) der Badewanne (2) befestigt ist, und die LED (4) ist in einer Hülse (8) auf der Außenseite der Badewanne (2) angeordnet, wobei die Hülse (8) zum Beispiel ebenfalls mit einer Verschraubung an der Badewanne (2) befestigbar ist. Die Abdichtung einer solchen Verschraubung (6) kann in bekannter Weise mit einem elastischen O-Ring erfolgen.

## Patentansprüche

1. Badewanne mit einem beleuchteten Innenraum, mit einer in der Wandung der Badewanne montierten Lichtquelle, **dadurch gekennzeichnet dass** die Lichtquelle (1) eine Licht-Emittierende-Diode (4), auch als LED bekannt, ist, und dass die LED (4) mehrere energetisch verschiedene lichtemittierende Halbleiterübergänge aufweist.

2. Badewanne nach Anspruch 1, **dadurch gekennzeichnet, daß** mehrere Lichtquellen (1) in der Wandung (7) der Badewanne (2) montiert sind.

3. Badewanne nach Anspruch 1 oder 2, **dadurch gekennzeichnet, daß** eine Lichtquelle (1) mit mehreren und/oder verschiedenen LEDs (4) versehen ist.

4. Badewanne nach einem der vorhergehenden Ansprüche, **dadurch gekennzeichnet, daß** die LED (4) lichtemittierende Halbleiterübergänge zur Erzeugung der drei spektralen Grundfarben rot, grün und blau aufweist, und sie Licht jeder Farbe des sichtbaren Spektrums ausstrahlen kann.

5. Badewanne nach einem der vorhergehenden Ansprüche, **dadurch gekennzeichnet, daß** die LEDs (4) in den Lichtquellen (1) einzeln und/oder gruppenweise steuerbar sind.

6. Badewanne nach einem der vorhergehenden Ansprüche, **dadurch gekennzeichnet, daß** die Lichtquellen (1) äquidistant auf dem Umfang der Badewanne (2) verteilt und/oder auf einer Geraden und/oder in geometrischen Mustern angeordnet sind.

7. Badewanne nach einem der vorhergehenden Ansprüche, **dadurch gekennzeichnet, daß** die LED (4) in einer Hülse (8), die mit einer Verschraubung an der Wandung (7) der Badewanne (2) befestigbar ist, angeordnet ist, und eine durchsichtige Scheibe (5) die LED (4) vom Innenraum der Badewanne (2) abtrennt.

8. Badewanne nach einem der vorhergehenden Ansprüche, **dadurch gekennzeichnet, daß** mehrere Lichtquellen (1) nahe beieinander in der Wandung (7) der Badewanne (2) montiert sind.

9. Badewanne nach einem der vorhergehenden Ansprüche, **dadurch gekennzeichnet, daß** eine mechanische Vorrichtung die LED (4) in der Lichtquelle (1) gegen eine andere LED (4) austauscht, insbesondere ein Revolverkopf.

10. Badewanne nach einem der vorhergehenden Ansprüche, **dadurch gekennzeichnet, daß** die Lichtquelle (1) großflächig ist, insbesondere streifen- und/oder bandförmig.

## Claims

1. Bathtub with an illuminated interior space, comprising a light source that is mounted in the wall of the bathtub, **characterised in that** the light source (1) is a light-emitting diode (4), also known as an LED, and **in that** the LED (4) has a plurality of energetically different light-emitting semiconductor junctions.

2. Bathtub according to claim 1, **characterised in that** a plurality of light sources (1) are mounted in the wall (7) of the bathtub (2).

3. Bathtub according to claim 1 or 2, **characterised in that** a light source (1) with a plurality of LEDs and/or with different LEDs (4) is provided.

4. Bathtub according to one of the preceding claims, **characterised in that** the LED (4) has light-emitting semiconductor junctions for generating the three fundamental spectral colours red, green and blue, and that it can emit light in every colour of the visible spectrum.

5. Bathtub according to one of the preceding claims, **characterised in that** the LEDs (4) in the light sources (1) can be controlled individually and/or in groups.

6. Bathtub according to one of the preceding claims, **characterised in that** the LEDs (4) in the light sources (1) are distributed equidistantly on the circumference of the bathtub (2) and/or are arranged on a straight line and/or in geometrical patterns.

7. Bathtub according to one of the preceding claims, **characterised in** the LED (4) is arranged in a sleeve (8), which can be fixed by means of a screw fastening on the wall (7) of the bathtub (2), and a transparent pane (5) separates the LED (4) from the interior space of the bathtub (2).

8. Bathtub according to one of the preceding claims, **characterised in that** a plurality of light sources (1) are mounted close together in the wall (7) of the bathtub (2).

9. Bathtub according to one of the preceding claims, a mechanical device, in particular a revolver head, exchanges the LED (4) in the light source (1) for another LED (4).

10. Bathtub according to one of the preceding claims, **characterised in that** the light source (1) has a large area, in particular is strip and/or band shaped.

## Revendications

1. Baignoire à intérieur éclairé, avec une source lumineuse montée dans la paroi de la baignoire, **caractérisée en ce que** la source lumineuse (1) est une diode électroluminescente (4), également appelée DEL, et **en ce que** la DEL (4) présente plusieurs jonctions semi-conductrices électroluminescentes qui sont différentes du point de vue énergétique.

2. Baignoire selon la revendication 1, **caractérisée en ce que** plusieurs sources lumineuses (1) sont montées dans la paroi (7) de la baignoire (2).

3. Baignoire selon la revendication 1 ou 2, **caractérisée en ce qu'**une source lumineuse (1) est pourvue de plusieurs DEL (4) et/ou de DEL (4) différentes.

4. Baignoire selon l'une des revendications précédentes, **caractérisée en ce que** la DEL (4) présente des jonctions semi-conductrices électroluminescentes pour produire les trois couleurs spectrales de base, rouge, vert et bleu, et elle peut émettre de la lumière de n'importe quelle couleur du spectre visible.

5. Baignoire selon l'une des revendications précédentes, **caractérisée en ce que** les DEL (4) des sources lumineuses (1) peuvent être commandées individuellement et/ou par groupes.

6. Baignoire selon l'une des revendications précédentes, **caractérisée en ce que** les sources lumineuses (1) sont réparties de manière équidistante sur le pourtour de la baignoire (2) et/ou sont disposées sur une droite et/ou en motifs géométriques.

7. Baignoire selon l'une des revendications précédentes, **caractérisée en ce que** la DEL (4) est disposée dans une douille (8) qui peut être fixée par un raccord à vis sur la paroi (7) de la baignoire (2), et une vitre transparente (5) sépare la DEL (4) de l'intérieur de la baignoire (2).

8. Baignoire selon l'une des revendications précédentes, **caractérisée en ce que** plusieurs sources lumineuses (1) sont disposées à proximité les unes des autres dans la paroi (7) de la baignoire (2).

9. Baignoire selon l'une des revendications précédentes, **caractérisée en ce qu'**un dispositif mécanique, en particulier une tête revolver, remplace la DEL (4) dans la source lumineuse (1) par une autre DEL (4).

10. Baignoire selon l'une des revendications précédentes, **caractérisée en ce que** la source lumineuse (1) est surfacique, en particulier en forme de ruban et/ou de bande.
